# EUROPEAN PATENT APPLICATION

(11) **EP 3 689 415 A1**
(43) Date of publication of application: **05.08.2020**
(21) Application number: 19154102.8
(22) Date of filing: 29.01.2019
(51) Int. Cl.: A61N 1/05, A61F 2/04, A61N 1/36, A61F 2/00

(54) **URINARY PUMPING DEVICE WITH AN IMPLANTABLE ACTIVATION ARRANGEMENT**

(71) Applicant: Universität Bern, 3012 Bern (CH)
(72) Inventor: Obrist, Dominik, 5034 Suhr (CH); Clavica, Francesco, 4552 Derendingen (CH); Burkhard, Fiona, 3014 Bern (CH); Schneider, Marc, 3612 Steffisburg (CH)
(74) Representative: Latscha Schöllhorn Partner AG

(57) **Abstract**

A urinary pumping device (1) for generating a fluid flow through an urethra (22; 220) of a patient (2; 20), comprises an implantable activation arrangement (11) and a control unit (12). The implantable activation arrangement (11) is configured to induce intermittent compression and release of the urethra (22) when being implanted into the patient (2). The control unit (12) is coupled to the activation arrangement (11). The control unit (12) is configured to operate the activation arrangement (11) such that the fluid flow through the urethra (22) is generated by the activation arrangement (11) inducing intermittent compression and release of the urethra (22).

## Description

### Technical Field

The present invention relates to a urinary pumping device according to the preamble of independent claim 1. Such devices can be used for generating a fluid flow through an urethra of a patient.

### Background Art

Today an increasing number of persons suffer from urological defects. Particularly, due to the increasing expectation of life in developed countries, urological dis-functions are getting more common. One kind of such urologic dis-functions relates to underactive bladders. Patients being confronted with an underactive bladder typically are not able to completely empty the bladder such that a residual portion of urine stays inside the bladder even after naturally emptying the bladder.

Such residual urine in the bladder may cause discomfort to the patient since urge to empty the bladder stays. It may also cause inflammations or other secondary diseases.

For treating patients having an underactive bladder it is known to introduce a pipe or catheter through the urethra into the bladder in order to remove the residual urine from the bladder. However, even though such process allows for sufficiently emptying the bladder it is comparably delicate and complicated to apply. In particular, introducing the pipe often is undesired and requires the operator or patient to be trained for not affecting the patient. Furthermore, such emptying of the bladder makes it necessary that appropriate equipment, which may be comparable bulky and/or heavy, is accessible. Additionally, using catheters may cause urinary infections.

Therefore, there is a need for an improved, easily applicable system or device allowing emptying the bladder of a patient.

### Disclosure of the Invention

According to the invention this need is settled by a urinary pumping device as it is defined by the features of independent claim 1. Preferred embodiments are subject of the dependent claims.

In particular, the invention is a urinary pumping device for generating a fluid flow through an urethra of a patient, which comprises an implantable activation arrangement and a control unit. The implantable activation arrangement is configured to induce intermittent compression and release of the urethra when being implanted into the patient. The control unit is coupled to the activation arrangement and configured to operate the activation arrangement such that the fluid flow through the urethra is generated by the activation arrangement inducing intermittent compression and release of the urethra.

By being capable of compressing and releasing the urethra, the device can operate as a peristaltic pump. Thereby, when being operated as a peristaltic pump the activation arrangement can be configured to compress and/or expand neighbouring sections of the urethra one after the other. Like this, the urine is pushed forward from the bladder into the urethra and outside through wave-like movements.

Preferably, the control unit is configured to operate the activation arrangement such as it works as an impedance pump. When being operated as impedance pump, the activation arrangement can be configured to compress and/or expand the urethra at specific frequency. Like this, a liquid or fluid can be pumped out of the bladder. Vice versa, a liquid or fluid can be pumped into the bladder by operating the impedance pump in an opposite direction by adapting frequency. Such filling of the bladder can be provided for therapeutic purposes. Also, an alternating filling and emptying of the bladder may be induced for therapeutic purposes.

The fluid to be pumped by the device can particularly be a liquid. More specifically, it can be urine to be pumped out of the bladder, a liquid medicament or another therapeutic liquid such as a saline solution to be pumped into the bladder.

The activation arrangement is to be implanted into the body of the patient in a surgical or invasive operation. When being implanted, the activation arrangement can be invisible and non-recognizable from outside the body. Such arrangement allows for a particular convenient application of the urinary pumping device. In particular, such application may be widely accepted by patients since the urinary pumping device disturbs the patient in everyday's life to a minimal extent only.

The term "inducing intermittent compression and release of the urethra" relates to a direct modification, i.e. compression and release, of the urethra as well as to an indirect modification. Direct modification can, e.g., be embodied by physically pressing the urethra or its neighbouring tissue. Indirect modification can, e.g., be embodied by activating a muscle such as the urethral sphincter which in turn compresses and releases the urethra.

The urinary pumping device according to the invention allows for providing a fluid out of or into the bladder. By being implanted into the body of the patient, acceptance of the device and, thus, of a treatment can be comparably high such that the quality of the treatment can be improved.

Preferably, the control unit is configured to control the activation arrangement such that a periodic compression and release of the urethra is generated by the activation arrangement at a predefined frequency and, optionally, a predefined amplitude, a predefined pressure, a predefined force and/or a predefined duty cycle. Such control unit allows for efficiently manipulate the activation arrangement such that it can be adjusted to the given situation and/or needs of the patient. Further, by predefining the frequency the impedance pumping can be implemented.

Thereby, the predefined frequency preferably is in a range of about 1 Hz to about 50 Hz and, particularly, in a range of about 5 Hz to about 20 Hz. Such frequency can be appropriate to induce impedance pumping for generating a flow of urine from the bladder to outside the body. Like this, the more or less complete content of the bladder can be delivered outside the body such that quasi no urine resides inside the bladder. Advantageously, the frequency is not constant over time but adapted as function of the flow or of the bladder volume. Such adaptation can be provided manually or automatically by the control unit.

Preferably, the activation arrangement comprises an implantable electrode configured to be implantable into the body of the patient such that the urethral sphincter of the patient is stimulatable by an electric field generated by the electrode of the activation arrangement. Stimulation of the urethral sphincter can particularly cause a contraction of the urethral sphincter. For creating a suitable electro magnetic field it can be appropriate that the activation arrangement comprises plural implantable electrodes. The electrode can comprise a coil or a similar structure. The electrode can be embodied or applied to directly stimulate the urethral sphincter or to indirectly stimulate the urethral sphincter via a nerve or the like.

Preferably, the activation arrangement comprises a surrounding portion dimensioned to be arranged around the urethra of the patient. Such surrounding portion can, e.g., be designed to be positioned or wrapped around the urethra. When being implanted, the surrounding portion can extend about the complete circumference of the urethra or a portion thereof. Such surrounding portion allows for efficiently acting on the urethra and thereby inducing pumping, e.g. as a peristaltic or impedance pump. Like this, the urinary pumping device can efficiently be designed and implemented.

Thereby, the activation arrangement preferably comprises a diameter adjuster configured to reduce a diameter of the surrounding portion when being arranged around the urethra of the patient. Such diameter adjuster may allow for efficiently pressurizing the urethra and thereby inducing a pumping either from or towards the bladder. The surrounding portion of the activation arrangement can have a recess or gap interrupting the surrounding portion in its circumference and the diameter adjuster of the activation arrangement can be configured to change the circumferential extension of the recess or gap. By changing the extension or dimension of the recess or gap, the diameter of the surrounding portion can be varied. For example, when reducing the circumferential extension of the recess the diameter of the surrounding portion is reduced correspondingly. Like this, the urethra can efficiently be pressurized.

For changing the dimension of the gap, the diameter adjuster of the activation arrangement can comprise a lever pivotably mounted to the surrounding portion at one side of the recess and connected to the other side of the recess. The term "pivotably" in this context can relate to a rotation about a specific angle or to a complete or 360° rotation. The lever can be connected to the other side of the recess by means of a band, cable, rod or any other suitable structure. By such pivotable lever the diameter of the surrounding portion can efficiently be varied. In particular, it can be varied in repetitive cycles such that intermittent compression and release of the urethra can efficiently be implemented.

Thereby, the diameter adjuster of the activation arrangement can comprise a drive coupled to the lever and configured to pivot the lever. Such drive can, e.g., have an electro-motor, a linear motor or the like. It allows for precisely and efficiently adjusting the diameter of the surrounding portion.

Preferably, the surrounding portion comprises a cuff or a cuff-like structure which can be configured to be positioned around the urethra. The surrounding portion preferably is embodied as or comprises a ring portion. In this context, the term "ring portion" covers sections of rings as well as complete or closed rings. Typically, the ring portion is designed to extend over a major part of the circumference of the urethra.

Preferably, the surrounding portion of the activation arrangement comprises a preferably polymeric dimension variable material which exhibits a change of a diameter of the surrounding portion when stimulated. Stimulation can be induced by any suitable and feasible means such as by temperature variation, radiation, pressure induction or the like. Such dimension variable material can allow for efficiently reducing the diameter of the surrounding portion and, thereby, pressurizing the urethra. The dimension variable material of the surrounding portion of the activation arrangement preferably is a polymeric material.

Thereby, the control unit preferably comprises a signal sender configured to send a stimulation signal to stimulate the dimension variable material of the surrounding portion of the activation arrangement. The stimulation signal can be any suitable signal, e.g., such as an electromagnetic signal, an ultrasonic signal or the like. Such stimulation via the signal sender allows the control unit to efficiently stimulate the dimension variable material of the surrounding portion and thereby adapt the dimension of the surrounding portion. For example, the dimension variable material can be configured to contract when receiving the stimulation signal from the signal sender of the control unit.

In another variant, the activation arrangement preferably comprises a pushing member, wherein the pushing member is configured to press the urethra of the patient. The pushing member can be any suitable structure such as a rod, a hammer or the like. Such pushing member allows for selectively pressurizing the urethra at a location where it can be efficiently compressed. Thereby, the urinary pumping device preferably comprises a drive coupled to the pushing member and configured to tilt the pushing member. Such drive allows for efficiently pressurizing the urethra.

Preferably, the urinary pumping device comprises a power supply unit configured to provide power to the activation arrangement. The power supply unit can be integral with other components of the device such as the activation arrangement or the control unit. Advantageously, the power supply unit is separate from the activation arrangement such that it is positionable remote from the activation arrangement. The power supply can, thus, be arranged distant from the activation arrangement such that the latter can be embodied comparably compact. Like this, the elements to be implanted can be comparably light weighted and small. This allows to increase patient's comfort when using the urinary pumping device such that also acceptance of the device can be increased.

The control unit can be integral with the other components of the urinary pumping device. Preferably, it is arranged to be separate from the activation arrangement or in a separate physical entity. When being a separate entity, the control unit can be connected to the activation arrangement or other components of the device by a wire or wirelessly. For a wireless communication, the activation arrangement preferably comprises a first wireless adapter and the control unit a second wireless adapter in communication with the first wireless adapter. Like this, the wirelessly connected control unit can be a remote control which may be beneficial for conveniently and efficiently operating the device. In particular, like this the body of the patient having the activation arrangement implanted can be completely healed and closed after implantation and operation can conveniently be controlled by a user or an operator.

Another aspect of the invention relates to a use of at least one electrode to generate a fluid flow through an urethra. The use comprises implanting the at least one electrode into a body of the patient such that a urethral sphincter of the patient is within an electric field generatable by the at least one electrode; and intermittently activating the at least one electrode such that the electric field is intermittently generated and the urethral sphincter is intermittently stimulated. Like this, a convenient and efficient pumping of fluid can be achieved.

### Brief Description of the Drawings

The urinary pumping device according to the invention is described in more detail herein below by way of exemplary embodiments and with reference to the attached schematic drawings, in which:
- Fig. 1: shows a schematic view of a first embodiment of a urinary pumping device according to the invention for a male patient; and
- Fig. 2: shows a schematic view of a second embodiment of a urinary pumping device according to the invention for a female patient.

### Description of Embodiments

In the following description certain terms are used for reasons of convenience and are not intended to limit the invention. The terms "right", "left", "up", "down", "under" and "above" refer to directions in the figures. The terminology comprises the explicitly mentioned terms as well as their derivations and terms with a similar meaning. Also, spatially relative terms, such as "beneath", "below", "lower", "above", "upper", "proximal", "distal", and the like, may be used to describe one element's or feature's relationship to another element or feature as illustrated in the figures. These spatially relative terms are intended to encompass different positions and orientations of the devices in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. The devices may be otherwise oriented (rotated 90 degrees or at other orientations), and the spatially relative descriptors used herein interpreted accordingly. Likewise, descriptions of movement along and around various axes include various special device positions and orientations.

To avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

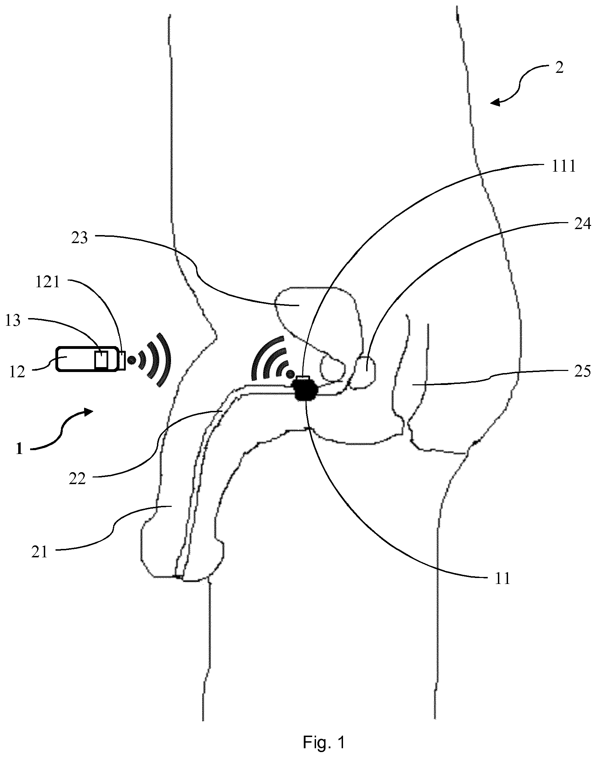

Fig. 1 shows a first embodiment of a urinary pumping device 1 applied to a male patient 2. The male patient 2 is schematically depicted such that some portions of inside his body are visible. In particular, a rectum 25, a bladder 23, a penis 21, an urethra 22 extending from the bladder 23 to an outlet of the penis 21, and a prostate 24 of the patient 2 are visible.

The urinary pumping device 1 comprises an activation arrangement with a cuff 11 as surrounding portion. The activation arrangement is implanted into the body of the patient 2 such that the cuff 11 is positioned around the urethra 22. The activation arrangement further has a first wireless adapter 111 mounted to the cuff 11. The urinary pumping device 1 further comprises a control unit 12 equipped with a second wireless adapter 121. The first and second wireless adapters 111, 121 are configured to establish a communication connection between the cuff 11 and the control unit 12. In particular, the control unit 12 is configured to transfer operation signals to the cuff 11 via the first and second wireless adapters 111, 121. The control unit 12 is equipped with a battery 13 as power supply unit. The battery 13 directly supplies energy to the components of the control unit 12 and indirectly supplies energy to the activation arrangement via induction.

The control unit 12 is configured to control the cuff 11 such that a periodic compression and release of the urethra 22 is generated by the cuff 11. More specifically, the control unit 12 induces the periodic compression and release of the urethra, wherein this compression and release is provided at a frequency in a range of about 5 Hz to about 20 Hz, at a predefined amplitude, a predefined duty cycle, a predefined force and a predefined pressure. These parameters can be adjusted by the patient 2 via the control unit 12. In this way, an impedance pump is implemented in the urethra 22 such that a flow of urine is induced from the bladder 23 to outside the patient's 2 body. In this way, the bladder 23 can be more or less completely emptied such that quasi no urine resides in the bladder 23.

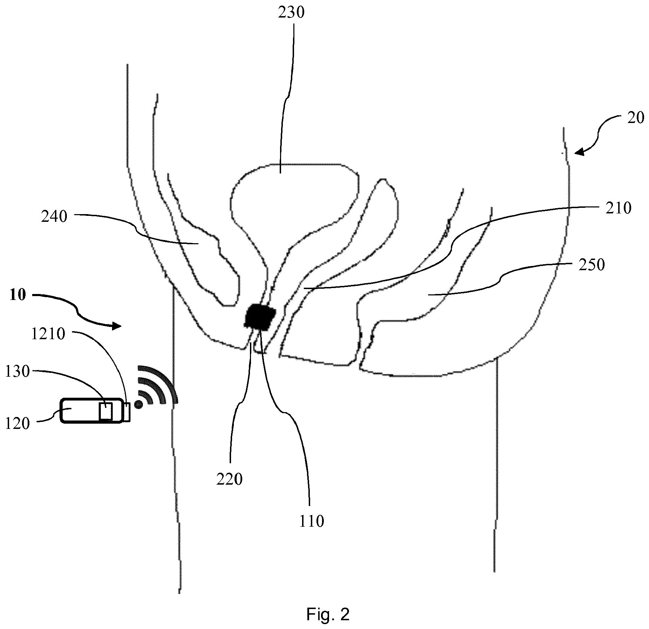

In Fig. 2 a second embodiment of a urinary pumping device 10 applied to a female patient 20 is shown. The female patient 20 is again schematically depicted such that some portions of inside her body are visible. In particular, a rectum 250, a bladder 230, a vagina 210, an urethra 220 extending from the bladder 230 to an outlet and a pubic bone 240 of the patient 20 are visible.

The urinary pumping device 10 comprises an activation arrangement with a cuff 110 as surrounding portion. The activation arrangement is implanted into the body of the patient 20 such that the cuff 110 is positioned around the urethra 220.

The urinary pumping device 10 further comprises a control unit 120 equipped with a wireless simulation signal sender 1210. The cuff 110 is made of a dimension variable polymeric material which can be stimulated. In particular, the cuff 11 is configured to reduce its diameter when receiving a stimulation signal from the control unit 120 via the simulation signal sender 1210. The simulation signal can, e.g., be a ultrasonic signal or the like. Like this, the control unit 120 can exhibit a change of a diameter of the cuff 110 by stimulating the material of the cuff 110.

The control unit 120 is equipped with a battery 130 as power supply unit. It further is configured to control the diameter of the cuff 110 such that a periodic compression and release of the urethra 220 is generated. More specifically, the control unit 120 induces the periodic compression and release of the urethra, wherein this compression and release is provided at a frequency in a range of about 5 Hz to about 20 Hz, at a predefined amplitude, a predefined duty cycle, a predefined force and a predefined pressure. In this way, an impedance pump is implemented in the urethra 220 such that a flow of urine is induced from the bladder 230 to outside the patient's 20 body. In this way, the bladder 230 can be more or less completely emptied such that quasi no urine resides in the bladder 230.

This description and the accompanying drawings that illustrate aspects and embodiments of the present invention should not be taken as limiting-the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the spirit and scope of this description and the claims. In some instances, well-known circuits, structures and techniques have not been shown in detail in order not to obscure the invention. Thus, it will be understood that changes and modifications may be made by those of ordinary skill within the scope and spirit of the following claims. In particular, the present invention covers further embodiments with any combination of features from different embodiments described above and below.

The disclosure also covers all further features shown in the Figs. individually although they may not have been described in the afore or following description. Also, single alternatives of the embodiments described in the figures and the description and single alternatives of features thereof can be disclaimed from the subject matter of the invention or from disclosed subject matter. The disclosure comprises subject matter consisting of the features defined in the claims or the exemplary embodiments as well as subject matter comprising said features.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

## Claims

1. A urinary pumping device (1; 10) for generating a fluid flow through an urethra (22; 220) of a patient (2; 20), **characterized by**
an implantable activation arrangement (11; 110) configured to induce intermittent compression and release of the urethra (22; 220) when being implanted into the patient (2; 20), and
a control unit (12; 120) coupled to the activation arrangement (11; 110), wherein
the control unit (12; 120) is configured to operate the activation arrangement (11; 110) such that the fluid flow through the urethra (22; 220) is generated by the activation arrangement (11; 110) inducing intermittent compression and release of the urethra (22; 220).

2. The urinary pumping device (1; 10) of claim 1, wherein the control unit (12; 120) is configured to operate the activation arrangement (11; 110) to work as an impedance pump.

3. The urinary pumping device (1; 10) of claim 1 or 2, wherein the control unit (12; 120) is configured to control the activation arrangement (11; 110) such that a periodic compression and release of the urethra (22; 220) is generated by the activation arrangement (11; 110) at a predefined frequency and, optionally, a predefined amplitude, a predefined pressure, a predefined force and/or a predefined duty cycle.

4. The urinary pumping device (1; 10) of claim 3, wherein the predefined frequency is in a range of about 1 Hz to about 50 Hz and, preferably, in a range of about 5 Hz to about 20 Hz.

5. The urinary pumping device (1; 10) of any one of the preceding claims, wherein activation arrangement (11; 110) comprises an implantable electrode configured to be implantable into the body of the patient (2; 20) such that the urethral sphincter of the patient (2; 20) is stimulatable by an electric field generated by the electrode of the activation arrangement (11; 110).

6. The urinary pumping device (1; 10) of any one of the preceding claims, wherein the activation arrangement (11; 110) comprises a surrounding portion (11; 110) dimensioned to be arranged around the urethra (22; 220) of the patient (2; 20).

7. The urinary pumping device (1; 10) of claim 6, wherein the surrounding portion (11; 110) comprises a cuff.

8. The urinary pumping device (1; 10) of 6 or 7, wherein the surrounding portion (11; 110) comprises a ring portion (117).

9. The urinary pumping device (1; 10) of any one of claims 6 to 8, wherein the activation arrangement (11; 110) comprises a diameter adjuster configured to reduce a diameter of the surrounding portion (11; 110) when being arranged around the urethra (22; 220) of the patient (2; 20).

10. The urinary pumping device (1; 10) of any one of claims 6 to 9, wherein the surrounding portion (11; 110) of the activation arrangement (11; 110) comprises a preferably polymeric dimension variable material which exhibits a change of a diameter of the surrounding portion (11; 110) when stimulated.

11. The urinary pumping device (1; 10) of claim 10, wherein the control unit comprises a signal sender (1210) configured to send a stimulation signal to stimulate the dimension variable material of the surrounding portion (11; 110) of the activation arrangement (11; 110).

12. The urinary pumping device (1; 10) of any one of the preceding claims, comprising a power supply unit (13; 130) configured to provide power to the activation arrangement (11; 110).

13. The urinary pumping device (1; 10) of any one of the preceding claims, wherein the control unit (12; 120) is arranged to be separate from the activation arrangement (11; 110) such that it is positionable remote from the activation arrangement (11; 110).

14. The urinary pumping device (1; 10) of claim 13, wherein the activation arrangement (11; 110) comprises a first wireless adapter and the control unit (12; 120) comprises a second wireless adapter in communication with the first wireless adapter.

15. The urinary pumping device (1; 10) of any one of the preceding, wherein the activation arrangement (11; 110) comprises a pushing member, and the pushing member is configured to compress the urethra (22; 220) of the patient (2; 20).
